# EUROPEAN PATENT APPLICATION

(11) **EP 4 265 636 A1**
(43) Date of publication of application: **25.10.2023**
(21) Application number: 22168872.4
(22) Date of filing: 19.04.2022
(51) Int. Cl.: C07K 14/79, C07K 7/06, C07K 14/50

(54) **PREPARATION OF TARGET PEPTIDES AND PROTEINS**

(71) Applicant: mk2 Biotechnologies GmbH, 85152 Planegg (DE)
(72) Inventor: WIESBECK, Patrick, 81925 Munich (DE); GIUMAN, Marco, 82140 Olching (DE)
(74) Representative: Kador & Partner Part mbB

(57) **Abstract**

The present invention relates to a fusion polypeptide comprising a purification domain; a cleavage domain; and a target peptide domain. The purification domain comprises 3 to 50 repeats of a nonapeptide, each nonapeptide independently having an amino acid sequence GX²X³X⁴X⁵X⁶X⁷X⁸X⁹, whereby X² may be G, N or D, X³ may be A, S, G, D, E, L or N, X⁴ may be G or A, X⁵ may be N, D, A or S, X⁶ may be D or N, X⁷ may be T, I, V or L, X⁸ may be L, V, I, F or Y, and X⁹ may be Y, I, V, F, T, N, D, K or S. The cleavage domain comprises an autoprotease or an amino acid sequence X^{a}SX^{b}X^{c}X^{d}X^{e}X^{f}X^{g}, whereby X^{a} is only optional and may be any amino acid, X^{b} may be H, R, S, N, Y, G, K or Q, X^{c} may be H, S or N, X^{d} may be T, S, L, M, G, N, Q or W, X^{e} is only optional may be S, P, A, T, L, R or D, X^{f} is only optional and may be L, P, E, G, T, A, F or S, and X^{g} is only optional and may be any amino acid. Further disclosed is a method for preparing a target peptide using the fusion polypeptide of the invention.

## Description

The present invention relates to fusion polypeptides, nucleic acid molecules encoding said fusion polypeptides and genetically modified cells including said nucleic acid molecules. Likewise, the present invention encompasses methods for preparing target peptides or proteins using said fusion polypeptides.

### Background

Recombinant peptides and proteins are widely used as functional components or active ingredients in the pharmaceutical industry, in cosmetics, in the food industry, agriculture, material research and medicine.

To allow for a simpler and quicker purification after recombinant overexpression, the peptides or proteins of interest are usually provided with a purification domain, i.e. the peptides or proteins of interest are overexpressed as fusion proteins, in which they are covalently linked to a purification domain. Conventional examples of such purification domains include chitin-binding domains, cellulose-binding domains, starch-binding domains, carbohydrate-binding domains, hexahistidine-tags, maltose-binding-protein tags, streptavidin tags, calmodulin-tags, FLAG-tags, etc. Most of the conventionally used purification domains exhibit a specific affinity to certain small molecule or protein ligands Accordingly, the purification after recombinant overexpression using such domains is regularly based on affinity chromatography, in which one specific small molecule or protein ligand is immobilized on a column material, such as Sepharose or Agarose, such that the corresponding purification domain may specifically bind to said ligand. Typical examples of such purification methods based on affinity chromatography are the hexahistidine tag with affinity to nickel-NTA, the streptavidin tag with affinity to streptavidin, or antibodies with affinity to protein A. One severe disadvantage of these methods is the costintensive production of the column materials (D. Guillén et al., Appl Microbiol Biotechnol 2013, 97, 4141-4148; Banki et al., Microbial Cell Factories 2005, 4, 32). Additionally, affinity chromatography is rather time-consuming.

A purification domain that overcomes this drawback is based on the natural occurring calcium-responsive repeats-in-toxin (RTX) motif that undergoes calcium-responsive, reversible precipitation (Hendrix et al., ACS Synth. Biol. 2014, 3, 969-971). Still, this purification domain has the disadvantage that it needs to be soluble and exhibit a specific conformation to enable calcium-responsive precipitation. Moreover, like other purification domains, also the application of this purification domain suffers from the drawback that it remains with the target peptide or protein after purification (Shur et al., BioTechniques 2013, 54, 197-206). However, authenticity of the recombinant peptides or proteins of interest is mandatory in the vast majority of applications, for example because the peptides are used for further coupling to other substances, or for regulatory reasons.

Hence, recombinant peptides and proteins of interest may only be prepared authentically if a cleavable domain is additionally included into the fusion protein. However, chemically induced cleavage, such as cyanogen bromide (CNBr) and 2-nitro-5-thiocyanobenzoic acid (NTCB) is unspecific and often toxic. Likewise, the inclusion of recognition sequences for external proteases requires the selection of specific external proteases, which are able to cleave the fusion protein in a manner such that no residues of the recognition sequence remain with the target peptide or protein of interest. Examples of such external proteases are the tobacco-etch-virus (TEV) protease and the SUMO (small ubiquitin-related modifier) protease. However, the use of external proteases tremendously increases the costs for the purification of recombinant peptides and proteins. In alternative, inteins or autoproteases may be included into the fusion protein, whereby no residues of the autoproteases may remain in the target peptide or protein if authenticity of the same is desired. The drawback of using inteins or autoproteases is that the cleavage is often cumbersome and/or inefficient. An example of an autoprotease, which enables the preparation of authentic peptides or proteins of interest, is the autoprotease N^{pro} of a pestivirus (WO 2006/113957). However, unintended activation of autoproteases during purification may lead to premature cleavage and thus, to loss in yield or the recombinant peptide or protein of interest. Therefore, when using autoproteases, the corresponding fusion protein regularly needs to be overexpressed in insoluble inclusion bodies, in which the autoprotease is kept inactive. This, in turn, considerably limits the selection of purification domains, since many of these domains must have an active conformation in order to efficiently and selectively bind to the corresponding ligand during affinity chromatography.

An alternative method for purifying recombinant peptides or proteins of interest without the application of purification domains is chromatography, in which the recombinant peptides or proteins are fractionated according to their size, electrostatic properties, or hydrophobicity. However, this requires detailed knowledge of these properties and is often non-specific due to co-eluted proteins. Another drawback is that the scale-up of such chromatographic purification of peptides and proteins of interest is often difficult and cost intense.

Hence, there is still a need for means and methods for the preparation of recombinant target peptides and proteins, which overcome the above disadvantages of the prior art. In particular, it is the object of the present invention to provide a fusion polypeptide that enables highly efficient purification together with highly specific and efficient protein cleavage for releasing the peptide or protein of interest, preferably having authentic termini. Accordingly, it is another object the present invention to provide methods for the preparation of a peptide or protein of interest, which enable a highly efficient purification by simple and robust operation together with a highly specific and efficient protein cleavage for releasing the peptide or protein of interest at high yields and at low overall costs.

### Summary of the invention

Upon intense research, the inventors of the present invention have surprisingly found that the above object is solved by a method for preparing a recombinant target peptide or protein applying a specific fusion polypeptide, which comprises a combination of a purification domain based on the natural calcium-responsive repeats-in-toxin (RTX) motif and a cleavage domain that enables efficient and selective intrinsic cleavage by autoproteolysis or by chemical protein cleavage for releasing the target peptide or protein.

Therefore, in one aspect, the present invention relates to a fusion polypeptide, comprising
i) a purification domain;
ii) a cleavage domain; and
iii) a target peptide domain;
   wherein the purification domain i) comprises 3 to 50 repeats of a nonapeptide, each nonapeptide independently having an amino acid sequence GX²X³X⁴X⁵X⁶X⁷X⁸X⁹, whereby X² may be G, N or D, X³ may be A, S, G, D, E, L or N, X⁴ may be G or A, X⁵ may be N, D, A or S, X⁶ may be D or N, X⁷ may be T, I, V or L, X⁸ may be L, V, I, F or Y, and X⁹ may be Y, I, V, F, T, N, D, K or S; and
   wherein the cleavage domain ii) comprises an autoprotease or an amino acid sequence X^{a}SX^{b}X^{c}X^{d}X^{e}X^{f}X^{g}, whereby X^{a} is only optional and may be any amino acid, X^{b} may be H, R, S, N, Y, G, K or Q, X^{c} may be H, S or N, X^{d} may be T, S, L, M, G, N, Q or W, X^{e} is only optional may be S, P, A, T, L, R or D, X^{f} is only optional and may be L, P, E, G, T, A, F or S, and X^{g} is only optional and may be any amino acid.

The fusion polypeptide of the present invention enables highly efficient purification by simple and robust operation together with highly specific and efficient protein cleavage for releasing the peptide or protein of interest, preferably having authentic termini.

In another aspect, the present invention refers to a recombinant nucleic acid molecule encoding the fusion polypeptide of the present invention.

Further, the present invention is directed to a host cell comprising the recombinant nucleic acid molecule of the present invention.

In yet another aspect, the present invention relates to methods for preparing a target peptide using the fusion polypeptide of the present invention.

Accordingly, the invention discloses a method for preparing a target peptide, comprising the steps of
a) providing a fusion polypeptide according to the invention;
b) precipitating the fusion polypeptide by adding Ca²⁺, preferably Ca²⁺-containing buffer;
c) cleaving the target peptide from the fusion protein; and
d) recovering the target peptide.

In the above method, the fusion polypeptide is first precipitated for purification and isolation and subsequently cleaved for releasing the target peptide.

In alternative, the present invention also encompasses a method for preparing a target peptide, comprising the steps of
a) providing a fusion polypeptide according to the invention, wherein the cleavage domain comprises the amino acid sequence X^{a}SX^{b}X^{c}X^{d}X^{e}X^{f}X^{g};
b) cleaving the target peptide from the fusion protein;
c) precipitation of the purification domain and cleavage domain by adding Ca²⁺, preferably Ca²⁺-containing buffer; and
d) recovering the target peptide.

In this alternative method, the fusion polypeptide is first cleaved for releasing the target peptide and, subsequently, the remaining purification domain and cleavage domain are removed by precipitation.

Both methods for the preparation of a peptide or protein of interest according to the present invention enable a highly efficient purification by simple and robust operation together with a highly specific and efficient protein cleavage for releasing the peptide or protein of interest. In particular, there is no need for one or more chromatographic purification steps. As a result, the peptide or protein of interest, preferably having authentic termini, can be provided in industrial scale at high yields and, simultaneously, at low overall costs.

### Figures

- Figure 1:: SDS-PAGE gel of purified lactoferricin (Lfc) prepared according to one embodiment of the methods of the present invention (Example 4).
- Figure 2:: SDS-PAGE gel and western blot illustrating the preparation of lactoferricin (Lfc) according to another embodiment of the methods of the present invention (Example 5).
- Figure 3:: SDS-PAGE gel of purified lactoferricin (Lfc) prepared according to another embodiment of the methods of the present invention (Example 6).
- Figure 4:: SDS-PAGE gel of purified basic fibroblast growth factor (FGF2) prepared according to one embodiment of the methods of the present invention (Example 7).
- Figure 5:: SDS-PAGE gel of purified lactoferricin (Lfc) prepared according to still another embodiment of the methods of the present invention (Example 8).
- Figure 6:: SDS-PAGE gel and western blot illustrating the preparation of basic fibroblast growth factor (FGF2) according to still another embodiment of the methods of the present invention (Example 9).

### Detailed description

### Fusion protein

In one aspect, the present invention refers to a fusion polypeptide, which may be equally denoted as fusionprotein, comprising
i) a purification domain,
ii) a cleavage domain; and
ii) a target peptide domain.

The purification domain of the fusion polypeptide according to the present invention is based on the natural calcium-responsive repeats-in-toxin (RTX) motif. Specifically, the fusion polypeptide comprises 3 to 50 repeats of a nonapeptide. Each nonapeptide in the purification domain independently has an amino acid sequence GX²X³X⁴X⁵X⁶X⁷X⁸X⁹, whereby X² may be G, N or D, X³ may be A, S, G, D, E, L or N, X⁴ may be G or A, X⁵ may be N, D, A or S, X⁶ may be D or N, X⁷ may be T, I, V or L, X⁸ may be L, V, I, F or Y, and X⁹ may be Y, I, V, F, T, N, D, K or S.

Accordingly, the glycine at position one of each nonapeptide, i.e. at the N-terminus thereof, is conserved whereas the amino acids at the positions two to nine in each nonapeptide may vary as outlined above. Likewise, each nonapeptide in the purification domain may have the same amino acid sequence. In this case, the nonapeptide repeats in the purification domain of the fusion polypeptide are preferably tandem repeats, i.e. direct repeats having the same sequence. In alternative, the individual nonapeptides may differ in their amino acid sequences.

For the sake of clarity, it is noted that in this specification, all amino acids are indicated by the single letter code (also denoted as one letter code).

Preferably, the purification domain of the fusion polypeptide according to the invention comprises 5 to 40 repeats of the nonapeptide, preferably 5 to 25, more preferably 6 to 20 and even more preferably 7 to 17 repeats. Most preferably, the purification domain of the fusion polypeptide according to the present invention comprises 7, 12 or 17 repeats of the nonapeptide.

Further, it is preferred that X² in each nonapeptide having the an amino acid sequence GX²X³X⁴X⁵X⁶X⁷X⁸X⁹ is G, X⁴ is G, X⁶ is D, and X⁸ is L, I or F while X³, X⁵, X⁷ and X⁹ may be amino acids as defined above. Furthermore, it is preferred that each nonapeptide in the purification domain has the same amino acid sequence. Further preferably, the repeats of the nonapeptide are tandem repeats. Most preferably, each nonapeptide in the tandem repeats of the purification domain of the fusion polypeptide according to the invention has the amino acid sequence GGAGNDTLY (SEQ ID NO: 1).

The purification domain of the fusion polypeptide according to the invention is based on the natural repeats-in-toxin (RTX) motif and is calcium-responsive. Specifically, the purification domain is intrinsically disordered in the absence of calcium and soluble in aqueous environments, i.e. in aqueous solutions or buffers. In the presence of calcium, however, the purification domain forms a β-roll structure. This leads to precipitation, preferably quantitative precipitation, of the purification domain. Accordingly, when the purification domain is comprised in the fusion polypeptide, the complete fusion polypeptide precipitates in the presence of calcium. Likewise, when the target peptide or protein has been cleaved off from the fusion polypeptide, the remaining purification domain and cleavage domain fragment precipitates in the presence of calcium. Hence, this allows for highly selective and efficient precipitation of those proteins and/or protein fragments that contain the purification domain according to the invention from a mixture of peptides and proteins. In this context, it is important to note that the precipitation of the fusion polypeptide is independent from the nature of the cleavage domain and from the nature of the respective target peptide or protein, like size, polarity, hydrophobicity and/or toxicity. Likewise, the fusion polypeptide or the remaining purification domain and cleavage domain fragment after cleavage may be selectively and efficiently precipitated under a broad variety of conditions under which the fusion polypeptide or said fragment is kept, e.g. temperature, pH, chaotropic or kosmotropic buffers, etc. Consequently, it is the advantage of the purification domain according to the present invention that it allows for the selective and efficient precipitation of virtually each and every fusion polypeptide containing the purification domain under a broad variety of process conditions. Moreover, it is important to note that the precipitation by calcium is reversible. Calcium-precipitated proteins and/or protein fragments containing the purification domain according to the invention, e.g. the fusion polypeptide, may be re-solubilized by adding agents that complex calcium, such as chelating agents like EDTA or EGTA. In alternative, calcium-precipitated proteins and/or protein fragments may be re-solubilized by repeated washing in suitable buffers without any calcium or with low calcium concentration to reduce the overall calcium concentration. Such suitable buffers may also include chaotropic buffers, i.e. buffers having high concentrations of chaotropic agents such as urea, thiourea, guanidinium thiocyanate and guanidinium hydrochloride. Of course, these procedures may also be combined.

For the sake of clarity, it is noted that throughout this description, the term *"calcium"* does not refer to elementary calcium but to Ca²⁺-ions. Accordingly, the term *"calcium-containing buffer"* relates to buffers containing Ca²⁺-ions, which are prepared by using calcium salts, such as CaCl₂, CaCO₃, Ca(OH)₂, Ca(NO₃)₂, CaSO₄ and the like.

Preferably, the purification domain according to the present invention comprises a capping sequence. A preferred capping sequence is a peptide having the amino acid sequence INAGADQLWF ARQGNDLEIR ILGTDDALTV HDWYRDADHR VEIIHAANQA VDQAGIEKLV EAMAQYPD (SEQ ID No: 2). The capping sequence may support the purification domain and increase its efficiency in calcium-responsive conformational change. In other words, the capping sequence may increase the efficiency in calcium-responsive precipitation of the purification domain. Further, the capping sequence may be N-terminal or C-terminal to the repeats of the nonapeptide in the purification domain according to the invention.

In general, although not falling under the scope of the claims, the purification domain according to the present invention may virtually be combined with each cleavable domain of the prior art, such as inteins; Sortase A (SrtA); FrpC protein; Cystein protease domain (CPD); combinations of recognition sequences or motifs and external endopeptidases, like Tobacco Etch Virus (TEV) protease, thrombin protease, SUMO protease, Factor Xa, HIV-1 Protease, preScission protease cleavage site, HCV Protease Cleavage Site, RecA Cleavage Site, or chemical cleavage sites in a fusionprotein further comprising a peptide or protein of interest. Put another way, i.e. the purification domain can be virtually combined with any conventional protein purification process of the prior art.

The cleavage domain of the fusion polypeptide according to the present invention either comprises an autoprotease or an amino acid sequence X^{a}SX^{b}X^{c}X^{d}X^{e}X^{f}X^{g}.

The term *"autoprotease"* refers to an intrinsic protease contained in the substrate to be cleaved, in the present invention the fusion polypeptide, which may catalyse the proteolysis of the substrate, here the fusion polypeptide, at a predetermined position.

In case the cleavage domain of the present invention comprises an autoprotease, the autoprotease is preferably a viral autoprotease, more preferably an autoprotease derived from a virus of the family *Flaviviridae,* even more preferably an autoprotease derived from a pestivirus, and still more preferably an N^{Pro} wildtype autoprotease or an active fragment or an active mutant thereof. N^{Pro} is an autoprotease with a length of 168 amino acids and an apparent Mᵣ of about 20,000 (in vivo).

An example of a suitable N^{Pro} wildtype autoprotease is the N^{Pro} autoprotease of the Classical Swine Fever Virus (CSFV), in particular of the CSFV strain Alfort (Gottipati et al., PLoS Pathogens 2013, 9, e1003704). Likewise, examples of suitable active mutants of the N^{Pro} autoprotease are disclosed in WO 2006/113957 A1, EP 1 874 932 B1, EP 1 874 933 B1, EP 2 366 719 B1, EP 2 746 390 A1 and EP 2 935 576 B1, all of which are incorporated herein by reference.

In the present invention, the autoprotease of the cleavage domain is preferably an N^{Pro} wildtype autoprotease derived from CSFV (SEQ ID No: 3) or an active mutant thereof, more preferably the mutant is CSFV N^{Pro} EDDIE (SEQ ID No: 4) or a Δ1-16 CSFV N^{Pro} EDDIE derivative (SEQ ID No: 5).

The N^{Pro} wildtype autoprotease derived from CSFV (SEQ ID No: 3) exhibits the following amino acid sequence:

MELNHFELLY KTSKQKPVGV EEPVYDTAGR PLFGNPSEVH PQSTLKLPHD
RGRGDIRTTL RDLPRKGDCR SGNHLGPVSG IYIKPGPVYY QDYTGPVYHR
APLEFFDEAQ FCEVTKRIGR VTGSDGKLYH IYVCVDGCIL LKLAKRGTPR
TLKWIRNFTN CPLWVTSC

The mutant is CSFV N^{Pro} EDDIE (SEQ ID No: 4) exhibits the following amino acid sequence:

MELNHFELLY KTSKQKPVGV EEPVYDTAGR PLFGNPSEVH PQSTLKLPHD
RGEDDIETTL RDLPRKGDCR SGNHLGPVSG IYIKPGPVYY QDYTGPVYHR
APLEFFDETQ FEETTKRIGR VTGSDGKLYH IYVEVDGEIL LKQAKRGTPR
TLKWTRNTTN CPLWVTSC

The Δ1-16 CSFV N^{Pro} EDDIE derivative (SEQ ID No: 5) exhibits the following amino acid sequence:

PVGVEEPVYD TAGRPLFGNP SEVHPQSTLK LPHDRGEDDI ETTLRDLPRK
GDCRSGNHLG PVSGIYIKPG PVYYQDYTGP VYHRAPLEFF DETQFEETTK
RIGRVTGSDG KLYHIYVEVD GEILLKQAKR GTPRTLKWTR NTTNCPLWVT SC

The cleavage domain comprising an autoprotease preferably cleaves the fusion polypeptide after the C-terminus of the autoprotease and before the N-terminus of the target peptide domain. More preferably, autoproteolytic cleavage occurs such that no amino acid residues of the autoprotease domain remain with the target peptide. As a result, a target peptide or protein having an authentic N-terminus is obtained, provided that the N-terminus of the target peptide domain is directly fused to the C-terminus of the autoprotease, which is especially preferred. Particularly the use of the preferred N^{Pro} wildtype autoprotease derived from CSFV (SEQ ID No: 3), of the preferred mutant CSFV N^{Pro} EDDIE (SEQ ID No: 4) or of the preferred Δ1-16 CSFV N^{Pro} EDDIE derivative (SEQ ID No: 5) allows for the preparation of peptides or proteins of interest having an authentic N-terminus as the autoproteolytic cleavage generally occurs after the last C-terminal amino acid in the sequence of said autoproteases (cysteine).

Owing to this, when the cleavage domain comprises an autoprotease, the target peptide domain is preferably C-terminally fused to the cleavage domain in the fusion polypeptide. Accordingly, the fusion polypeptide according to the invention preferably comprises, in direction from the N-terminus to the C-terminus, i) the purification domain, optionally including the capping sequence; ii) the cleavage domain comprising the autoprotease; and iii) the target peptide domain.

It is important to note that autoproteases including the autoprotease of the present invention require an active state for autoproteolysis, i.e. they require at least partially proper folding into an active conformation. Accordingly, as soon as such an active state is reached, e.g. already during the recombinant overexpression of a fusion protein *in vivo* or during preparation of a target peptide from a fusion protein containing an autoprotease *in vitro,* the autoproteolysis starts. However, unintended activation of the autoprotease in the cleavage domain leads to premature cleavage and, thus, to lower yields of the peptide or protein of interest. Therefore, when using a cleavage domain comprising an autoprotease, the corresponding fusion polypeptide is regularly overexpressed in the form of inclusion bodies (IBs). As used herein, IBs represent insoluble, dense protein aggregates mainly containing the overexpressed fusion polypeptide in an inactive state. Likewise, the obtained fusion polypeptide is re-solubilised and kept in chaotropic buffers in an inactive state. Only, when autoproteolysis is intended, the fusion polypeptide is refolded into an active state.

In alternative, the cleavage domain of the present invention comprises an amino acid sequence X^{a}SX^{b}X^{c}X^{d}X^{e}X^{f}X^{g}, wherein X^{a} is only optional and may be any amino acid, X^{b} may be H, R, S, N, Y, G, K or Q, X^{c} may be H, S or N, X^{d} may be T, S, L, M, G, N, Q or W, X^{e} is only optional may be S, P, A, T, L, R or D, X^{f} is only optional and may be L, P, E, G, T, A, F or S, and X^{g} is only optional and may be any amino acid.

The above amino acid sequence X^{a}SX^{b}X^{c}X^{d}X^{e}X^{f}X^{g} enables the highly efficient and selective chemically induced cleavage of the sequence before the conserved serine residue, i.e. N-terminal thereof, in the presence of nickel. Accordingly, this type of cleavage may be denoted as sequence-specific nickel-assisted cleavage (SNAC) (Dang et al., Nature Methods 2019, 16, 319-322; WO2007/091907 A1).

Preferably, X^{a} in the amino acid sequence X^{a}SX^{b}X^{c}X^{d}X^{e}X^{f}X^{g} is G, X^{b} is H or R, X^{c} is H, X^{d} is W, X^{e} is A, and X^{f} is P while X^{a} and X^{g} may be amino acids as defined above. More preferably, the amino acid sequence X^{a}SX^{b}X^{c}X^{d}X^{e}X^{f}X^{g} is SRHWAP (SEQ ID No: 6) or GSHHW (SEQ ID No: 7). Most preferably, the nickel-assisted cleavable amino acid sequence in the cleavage domain of the fusion polypeptide according to the invention has the amino acid sequence SRHWAP (SEQ ID NO: 6).

The cleavage domain comprising the amino acid sequence X^{a}SX^{b}X^{c}X^{d}X^{e}X^{f}X^{g} preferably cleaves the fusion polypeptide after the C-terminus of the target peptide domain and N-terminally of the serine residue of said sequence. More preferably, nickel-induced cleavage occurs such that no amino acid residues of the amino acid sequence X^{a}SX^{b}X^{c}X^{d}X^{e}X^{f}X^{g} remain with the target peptide. As a result, a target peptide or protein having an authentic C-terminus is obtained. Particularly the use of the preferred amino acid sequence SRHWAP (SEQ ID NO: 6) allows for the preparation of peptides or proteins of interest having an authentic C-terminus.

Owing to this, when the cleavage domain comprises the nickel-assisted cleavable amino acid sequence X^{a}SX^{b}X^{c}X^{d}X^{e}X^{f}X^{g}, the target peptide domain is preferably fused directly to the N-terminus of the cleavage domain in the fusion polypeptide. Accordingly, the fusion polypeptide according to the invention comprises, in direction from the N-terminus to the C-terminus, iii) the target peptide domain, ii) the cleavage domain comprising the amino acid sequence X^{a}SX^{b}X^{c}X^{d}X^{e}X^{f}X^{g} and i) the purification domain, optionally including the capping sequence.

As used herein, the term *"nickel"* does not refer to elementary nickel but to Ni²⁺-ions. Accordingly, the term *"nickel-containing buffer"* relates to buffers containing Ni²⁺-ions, which are prepared by using nickel salts, such as NiCl₂, NiCO₃, Ni(OH)₂, Ni(NO₃)₂, NiSO₄ and the like.

In this context, it is important to note that the cleavage efficiency of the cleavage domain of the fusion polypeptide comprising the above nickel-assisted cleavable amino acid sequence X^{a}SX^{b}X^{c}X^{d}X^{e}X^{f}X^{g} is independent from the nature of the purification domain and from the nature of the respective target peptide or protein, like size, polarity, hydrophobicity and/or toxicity. Likewise, the fusion polypeptide may be selectively and efficiently cleaved in the presence of nickel under a broad variety of conditions under which the fusion polypeptide is kept, e.g. temperature, pH, chaotropic or kosmotropic buffers, etc. Consequently, it is the advantage of the cleavage domain comprising the amino acid sequence X^{a}SX^{b}X^{c}X^{d}X^{e}X^{f}X^{g} according to the present invention that it allows for the selective and efficient cleavage of virtually each and every fusion polypeptide containing said cleavage domain under a broad variety of process conditions.

Hence, although not falling under the scope of the claims, the cleavage domain comprising the amino acid sequence X^{a}SX^{b}X^{c}X^{d}X^{e}X^{f}X^{g} according to the present invention may virtually be combined with each conventional purification domain or tag of the prior art, like chitin-binding domains, cellulose-binding domains, starch-binding domains, carbohydrate-binding domains, poly-His-tags, maltose-binding-protein tags, streptavidin tags, calmodulin-tags, FLAG-tags, GST-Tags, Myc-Tag, Poly Arginin, Poly Aspartate, Poly Cystein, Halo-Tag, Protein A-Tag, Protein G-Tag, SUMO-Tag, in a fusionprotein further comprising a peptide or protein of interest. In other words, the cleavage domain comprising the amino acid sequence X^{a}SX^{b}X^{c}X^{d}X^{e}X^{f}X^{g} according to the present invention can be combined with any conventional protein purification process of the prior art.

The target peptide domain of the present invention is not particularly limited and may comprise virtually any peptide or protein of interest independent of its nature, such as length, polarity, hydrophobicity and/or toxicity.

Accordingly, target peptide domain may comprise peptide sequences of two or more amino acids in length, e.g. of from 2-2000 or more amino acids, from 2 to 1000 amino acids, from 2 to 500 amino acids or from 2 to 250 amino acids.

### Recombinant nucleic acid molecule and host cell

In a further aspect, the present invention relates to a recombinant nucleic acid molecule encoding the fusion polypeptide according to the invention.

The recombinant nucleic acid molecule may be present in single-stranded or double-stranded form, e.g. as RNA or DNA. Preferably, the nucleic acid molecule is a double-stranded DNA molecule. Optionally, the nucleic acid sequence encoding the fusion polypeptide is operatively linked to an expression control sequence, e.g. to a promoter and/or enhancer, i.e. a sequence that enables expression in a host cell. For example, the expression control sequence can comprise an autoinducible, chemically and/or thermally inducible promoter, which allows for a targeted control of expression.

Preferably, the recombinant nucleic acid molecule is arranged on a vector, i.e. a nucleic acid construct, which may be introduced in a host cell. Exemplary vectors are viral vectors, plasmids and cosmids suitable for the introduction in a prokaryotic or eukaryotic host cell. More preferably, the vector is a plasmid, i.e. the recombinant nucleic acid molecule is arranged in a plasmid, in particular a plasmid suitable for the introduction in a prokaryotic host cell.

Optionally, the nucleic acid molecule encoding the fusion polypeptide comprises a signal peptide encoding sequence controlling the type of fusion polypeptide expression in the host cell. Preferably, a signal peptide encoding sequence controlling expression in the form of insoluble inclusion bodies in the host cell is present.

The present invention further relates to a host cell comprising the recombinant nucleic acid molecule as defined above. The host cell according to the invention preferably includes the recombinant nucleic acid molecule arranged on a vector and is, preferably, able to express the fusion polypeptide of the invention, in the form of insoluble inclusion bodies or in soluble form. The host cell may be a prokaryotic or eukaryotic cell, e.g. a gram-negative bacterial cell, such as an *E. coli* cell, or a gram-positive bacterial cell, such as a *Bacillus subtilis* or *Bacillus licheniformis* cell. On the other hand, the cell may also be a eukaryotic cell, for example a yeast cell, such as a *Pichia Pastoris* or a *Saccharomyces cerevisiae* cell, an insect cell or a mammal cell, such as a Chinese hamster ovary (CHO) cell or a human embryonic kidney (HEK) cell.

### Methods for the preparation of target peptides and proteins

In yet another aspect, the present invention relates to methods for preparing a target peptide or protein using the fusion polypeptide according to the present invention. Consequently, each embodiment as described above for the fusion polypeptide of the invention equally applies to the methods of the present invention using said fusion polypeptide.

A first method for preparing a target peptide or protein according to the invention comprises the steps of
a) providing a fusion polypeptide according to the invention by recombinant expression;
b) precipitating the fusion polypeptide by adding Ca²⁺;
c) cleaving the target peptide from the fusion protein; and
d) recovering the target peptide.

In this method, the fusion polypeptide is first precipitated for purification and isolation and subsequently cleaved for releasing the target peptide.

In step a) of the first method, a fusion polypeptide according to the invention is provided by recombinant expression. This comprises providing a host cell expressing the fusion polypeptide. Such a host cell is obtainable by introducing a nucleic acid molecule including a sequence encoding the fusion polypeptide, preferably in the form of a vector, into the cell by known methods, such as transfection or transformation.

The host cell is cultured in a suitable culture medium, e.g. in a culture medium conventionally used for the respective cell type. Culturing is conducted under conditions, wherein expression of the fusion polypeptide is enabled. For instance, an inducible promoter, such as an auto-inducible, chemically or thermally inducible promoter, may be used to control the expression of the fusion polypeptide.

In step b) of the first method, the fusion polypeptide is precipitated by adding Ca²⁺. As used herein, the term *"adding Ca²⁺"* encompasses contacting the fusion polypeptide according to the invention with Ca²⁺. This causes the calcium-responsive purification domain to form a β-roll structure leading to precipitation of the purification domain and of the complete fusion polypeptide.

Preferably, the fusion polypeptide is precipitated in step b) by adding Ca²⁺-containing buffer, i.e. by contacting the fusion polypeptide according to the invention with Ca²⁺-containing buffer, such as buffer containing CaCl₂, CaCO₃, Ca(OH)₂, Ca(NO₃)₂or CaSO₄.

The Ca²⁺-concentration in step b) preferably ranges from 25 to 500 mM, more preferably from 50 to 300 mM, even more preferably from 75 to 250 mM, and most preferably from 75 to 200 mM. Accordingly, it is preferred that after the addition of the Ca²⁺-containing buffer, the reaction mixture exhibits a Ca²⁺-concentration of from 25 to 500 mM, more preferably of from 50 to 300 mM, even more preferably of from 75 to 250 mM, and most preferably of rom 75 to 200 mM. The further composition of the Ca²⁺-containing buffer is not particularly limited as long as it contains a sufficient amount of Ca²⁺ for precipitating the fusion polypeptide as indicated above. An example of a preferred Ca²⁺-containing buffer for precipitating the fusion protein in step b) is an aqueous buffer containing 100 mM CaCl₂, 122 mM citric acid, 38 mM Na₂HPO₄, at a pH of 4.0. Another preferred Ca²⁺-containing buffer for precipitating the fusion protein in step b) is an aqueous buffer containing 100 mM CaCl₂, 1 M Tris base, 250 mM sucrose, 10 mM monothioglycerol, pH 8.2.

The incubation time of the precipitation step b) preferably ranges from 1 min to 16 hours, more preferably from 1 min to 8 hours, even more preferably from 1 to 90 min, still more preferably from 1 to 60 min, and most preferably from 5 to 45 min. Accordingly, precipitation step b) is not time consuming but rather quick and cost efficient as no chromatographic purification is required. In addition, the precipitation of the fusion polypeptide is independent from the nature of the cleavage domain and from the nature of the respective target peptide or protein, like size, polarity, hydrophobicity and/or toxicity. Accordingly, step b) of the first method according to the invention allows for highly selective and efficient precipitation of those fusionproteins that contain the purification domain according to the invention from a mixture of peptides and proteins.

Preferably, the fusion polypeptide precipitated in step b) is pelletized by centrifugation. Further preferably, the pelletized fusion polypeptide is washed one or more times by re-supending the pellet in a suitable washing buffer followed by another centrifugation for pelletizing. The buffer for washing the pelletized fusion polypeptide is not particularly limited as long as it does not re-solubilise the precipitated fusion polypeptide. An example of a preferred washing buffer is an aqueous buffer containing 100 mM HEPES, 25 mM CaCl₂ and 10 mM thioglycerol at a pH of 8.2. Another example of a preferred washing buffer is an aqueous buffer containing 1 M Tris-HCI, 250 mM sucrose, 25 mM CaCl₂, 2mM EDTA and 5 to 20 mM thioglycerol at a pH of 8.2

In step c) of the first method, the target peptide or protein is cleaved from the fusion polypeptide. Cleavage is either effected by autoproteolysis in a fusion protein comprising a cleavage domain that includes an autoprotease or chemically induced in the presence of nickel in a fusion protein comprising a cleavage domain that includes a nickel-assisted cleavable amino acid sequence X^{a}SX^{b}X^{c}X^{d}X^{e}X^{f}X^{g} as defined above, respectively.

Autoproteolysis requires that the autoprotease comprised in the cleavage domain is in an active state, i.e. it must be at least partially folded and in an active conformation. Consequently, in case the cleavage in step c) is effected via autoproteolysis, the precipitated fusion polypeptide obtained in step b) is re-solubilised such that the autoprotease is converted into an active state. This is preferably accomplished by adding agents that complex Ca²⁺, such as chelating agents like EDTA or EGTA or by repeated washing in suitable buffers without any Ca²⁺ or with rather low Ca²⁺-concentration to reduce the overall Ca²⁺-concentration. As a result, the purification domain of the fusion polypeptide, becomes intrinsically disordered in the absence of calcium and again soluble in aqueous environments. More preferably, re-solubilisation of the precipitated fusion polypeptide obtained in step b) is accomplished by adding suitable aqueous buffers, which contain chelating agents like EDTA or EGTA to the pellet of the precipitated fusion polypeptide. Such re-solubilisation buffers are not particularly limited but need to contain chelating agents like EDTA or EGTA in amounts sufficient for the complete re-solubilisation of the precipitated fusion polypeptide and must provide conditions under which the autoprotease is active. An example of a preferred re-solubilisation buffer is an aqueous buffer comprising 50 mM EDTA, 1 M Tris-HCI, 250 mM sucrose, and 5 to 20 mM monothioglycerol at a pH of 8.2.

After re-solubilisation, the fusion polypeptide is preferably incubated to enable autoproteolysis. The incubation time after re-solubilising the precipitated fusion polypeptide for cleaving the target peptide domain from the fusion polypeptide by autoproteolysis preferably ranges from 8 to 70 hours, more preferably from 16 to 60 hours, and most preferably from 16 to 48 hours. Preferably, quantitative cleavage is obtained by autoproteolysis after the incubation time. The incubation temperature is not particularly limited in this step and may range from 0 to 40°C, preferably from 10 to 35°C and more preferably from 15 to 25°C, in particular 18°C.

An advantage of using a cleavage domain comprising an autoprotease is that peptides or proteins of interest having an authentic N-terminus may be prepared. Particularly the use of the preferred N^{Pro} wildtype autoprotease derived from CSFV (SEQ ID No: 3), of the preferred mutant is CSFV N^{Pro} EDDIE (SEQ ID No: 4) or of the preferred Δ1-16 CSFV N^{Pro} EDDIE derivative (SEQ ID No: 5) as the autoprotease allows for the preparation of peptides or proteins of interest having an authentic N-terminus as the autoproteolytic cleavage generally occurs after the last C-terminal amino acid in the sequence of said autoproteases. Hence, provided that the N-terminus of the target peptide domain is directly fused to the C-terminus of the autoprotease, which is especially preferred, an authentic N-terminus of the target peptide domain is obtained, i.e. no amino acid residues of the cleavage domain or of any linker remain with the target peptide domain.

In alternative, cleavage of the target peptide or protein off from the fusion polypeptide in step c) of the first method according to the invention is chemically induced in the presence of nickel in a fusion protein comprising a cleavage domain that includes a nickel-assisted cleavable amino acid sequence X^{a}SX^{b}X^{c}X^{d}X^{e}X^{f}X^{g} as defined above. As disclosed above for the fusion polypeptide of the present invention, the amino acid sequence X^{a}SX^{b}X^{c}X^{d}X^{e}X^{f}X^{g} enables the highly efficient and selective chemically induced cleavage of the sequence before the conserved serine residue in the presence of Ni²⁺.

Notably, the nickel-assisted cleavable amino acid sequence X^{a}SX^{b}X^{c}X^{d}X^{e}X^{f}X^{g} does not require any specific active conformation or the like but cleavage is chemically induced in the presence of Ni²⁺. Accordingly, the nickel-assisted cleavage in step c) does not require that the precipitated fusion polypeptide obtained in step b) is re-solubilised. Rather, it is sufficient to re-suspend the pellet of the precipitated fusion polypeptide obtained in step b) in Ni²⁺-containing buffer to initiate selective and efficient cleavage of the fusion polypeptide.

The Ni²⁺-concentration in step c) preferably ranges from 0.1 to 25 mM, more preferably from 0.5 to 15 mM, even more preferably from 0.5 to 10 mM, and most preferably from 1 to 5 mM, in particular 3 mM. Accordingly, it is preferred that the applied Ni²⁺-containing buffer exhibits a Ni²⁺-concentration of from 0.1 to 25 mM, more preferably from 0.5 to 15 mM, even more preferably from 0.5 to 10 mM, and most preferably from 1 to 5 mM, in particular 3 mM. The further composition of the Ni²⁺-containing buffer is not particularly limited as long as it contains a sufficient amount of Ni²⁺ for efficient cleavage of the fusion polypeptide as indicated above. An example of a preferred Ni²⁺-containing buffer for re-suspending the fusion protein in step c) is an aqueous buffer containing 3 mM NiSO₄ and 100 mM HEPES at a pH of 8.2.

Optionally, the precipitated fusion polypeptide obtained in step b) may also be re-solubilised by repeated washing in Ni²⁺-containing buffer without any Ca²⁺ or with rather low Ca²⁺-concentration to reduce the overall Ca²⁺-concentration. However, the use of chelating agents like EDTA or EGTA for reducing the overall Ca²⁺-concentration is to be avoided as said chelating agents would also complex Ni²⁺, which would be detrimental for an efficient cleavage. For the sake of clarity, it is noted that in case the precipitated fusion polypeptide obtained in step b) is re-suspended in Ni²⁺-containing buffer, at least a part of the precipitated fusion polypeptide inevitably dissolves in the buffer. Accordingly, re-suspending the precipitated fusion polypeptide in Ni²⁺-containing buffer encompasses at least partially re-solubilising the precipitated fusion polypeptide in Ni²⁺-containing buffer in this context.

After re-suspension, the fusion polypeptide is preferably incubated for further cleavage. The incubation time after re-suspending the precipitated fusion polypeptide for cleaving the target peptide domain from the fusion polypeptide by the nickel-assisted cleavage preferably ranges from 10 min to 40 hours, more preferably from 5 to 35 hours, even more preferably from 5 to 24 hours and most preferably from 6 to 16 hours. Preferably, quantitative cleavage is obtained by nickel-assisted cleavage after the incubation time. The incubation temperature is not particularly limited in this step and may range from 15 to 85°C, preferably from 30 to 60°C and more preferably from 40 to 55°C.

As indicated above, it is an advantage of using a cleavage domain comprising a nickel-assisted cleavable amino acid sequence X^{a}SX^{b}X^{c}X^{d}X^{e}X^{f}X^{g} that the fusion polypeptide does not need to be soluble in the Ni²⁺-containing buffer. Hence, even when only re-suspended in the Ni²⁺-containing buffer, robust, highly selective and efficient cleavage of the fusion polypeptide is obtained. In addition, the cleavage is accomplished rather quick and at low costs. Another advantage is that peptides or proteins of interest having an authentic C-terminus may be prepared. Particularly the use of the preferred the amino acid sequence SRHWAP (SEQ ID No: 6) allows for the preparation of peptides or proteins of interest having an authentic C-terminus as the cleavage generally occurs before the conserved serine residue. Hence, provided that the C-terminus of the target peptide domain is directly fused to the N-terminus of the amino acid sequence SRHWAP, which is especially preferred, an authentic C-terminus of the target peptide domain is obtained, i.e. no amino acid residues of the cleavage domain or of any linker remain with the target peptide domain. An authentic N-terminus of the target peptide domain may simultaneously be obtained by an appropriate selection of the expression system for the fusion polypeptide, particularly of the host cell.

In step d) of the first method according to the invention, the target peptide, which has been cleaved off from the fusion polypeptide, is recovered.

For this purpose, step d) preferably comprises precipitating the purification domain and cleavage domain by adding Ca²⁺, either in the form of calcium salts or in the form of Ca²⁺-containing buffer.

Thereby, the Ca²⁺-concentration to be set may be the same as or different from the Ca²⁺-concentration applied in step b). Preferably, the Ca²⁺-concentration in step d) ranges from 25 to 500 mM, more preferably from 25 to 300 mM, even more preferably from 50 to 250 mM, and most preferably from 75 to 200 mM. Likewise, the incubation time may be the same or different from that applied in step b) and preferably ranges from 1 min to 16 hours, more preferably from 1 min to 8 hours, even more preferably from 1 to 90 min, still more preferably from 1 to 60 min, and most preferably from 5 to 45 min.

The precipitated purification domain and cleavage domain fragment is preferably pelletized by centrifugation and, thus, separated from the target peptide or protein, which remains in the supernatant.

Optionally, step d) may comprise additional steps for purifying and isolating the target peptide. Such additional steps comprise conventional measures known in the art, such as heat treatment, salting out, isoelectric precipitation, trichloroacetic acid (TCA) precipitation, ammonium sulphate precipitation, solvent precipitation and mixtures thereof. A chromatographic purification step, however, is not necessary for the recovery of the target peptide or protein in high yield and high purity, which renders the method for the preparation thereof less time consuming and less expensive.

In a preferred embodiment of the first method for preparing a target peptide or protein according to the invention, where a fusion polypeptide with a cleavage domain comprising an autoprotease is used, step a) includes the sub-steps of
a1) providing a fusion polypeptide comprising a cleavage domain, which comprises an autoprotease as described above, by recombinant expression in inclusion bodies (IBs), a2) solubilising the inclusion bodies, and
a3) refolding the fusion polypeptide.

Sub-step a1) is preferred because the autoprotease in the fusion polypeptide is in inactive state due to aggregation of the fusion polypeptide in the insoluble IBs, such that premature cleavage is avoided. In sub-step a1), the IBs are preferably obtained by lysis of the host cells after culturing, pelletizing via centrifugation, re-suspending the IB pellet in suitable aqueous washing buffers (e.g. containing 1% Triton or 1 M NaCl) or ddH₂O and another pelletizing via centrifugation. More preferably, re-suspending the IB pellet in suitable aqueous washing buffers (e.g. containing 1% Triton or 1 M NaCl) or ddH₂O and subsequent pelletizing via centrifugation is repeated one or more times for removing other cellular components, wherein the applied washing buffers may vary.

In sub-step a2), the IBs, preferably in the form of a pellet after washing as outlined above, are solubilised by methods known in the art. Preferably, the IBs are solubilized in suitable chaotropic buffers, i.e. aqueous buffers having high concentrations of chaotropic agents such as urea, thiourea, guanidinium thiocyanate and guanidinium chloride/guanidinium hydrochloride or mixtures thereof, optionally including reducing agents to abolish false formed disulfide bonds in the fusion polypeptide. A preferred example of a suitable chaotropic buffer is an aqueous buffer containing 6 M guanidinium chloride/guanidinium hydrochloride and 100 mM HEPES at a pH of 8.2. In chaotropic buffers, the fusion polypeptide comprising the autoprotease is denatured, i.e. essentially unfolded, and, hence, kept in an inactive state.

In sub-step a3), the solubilized fusion polypeptide is refolded by methods known in the art. Specifically, when the fusion polypeptide is solubilized in suitable chaotropic buffers in sub-step a2), the solubilized fusion polypeptide is preferably refolded by lowering the concentration of the chaotropic agents by dilution. Preferably, suitable kosmotropic buffers are used for dilution, such as aqueous buffers containing no or low concentrations of chaotropic agents. The temperature in sub-step a3) is preferably between -10 and 40°C, more preferably 0 to 30°C, in particular 10 to 25°C. The pH may broadly vary in sub-step a3), it may be from 3 to 12, from 4 to 11, from 5 to 10 or from 6 to 9. A preferred example of a kosmotropic buffer suitable for the refolding of the fusion protein is an aqueous buffer containing 1 M Tris-HCI, 250 mM sucrose, 2mM EDTA, 5 to 20 mM monothioglycerol at a pH of 8.2. Optionally, arginine may be added to the suitable kosmotropic buffers in concentrations known in the art (e.g. up to 1.0 M, preferably 0.4-0.6 M) for assisting the refolding of the fusion polypeptide. Upon refolding of the inactive fusion polypeptide in sub-step a3), the cleavage domain comprising the autoprotease is converted to an active state, i.e. it is renatured into an active conformation, such that cleavage of the fusion polypeptide via autoproteolysis starts.

This, however, may be interrupted instantly by precipitating the fusion polypeptide by adding Ca²⁺ in step b) of the first method as described above. Thereby, loss of target peptide or protein, which can no longer be recovered easily (due to the absence of a purification domain) is significantly reduced such that the overall yield of the peptide or protein of interest is not considerably limited. The precipitated fusion polypeptide is then pelletized by centrifugation.

In this embodiment, the cleavage step c) comprises the sub-steps of
c1) re-solubilising the precipitated fusion polypeptide obtained in step b), and
c2) incubating the re-solubilised fusion polypeptide for 8 to 70 hours, for cleaving the target peptide from the fusion protein by autoproteolysis

Re-solubilisation in sub-step c1) may be accomplished as outlined above, i.e. by adding chelating agents like EDTA or EGTA that complex Ca²⁺ to the pellet of the precipitated fusion polypeptide or by repeated washing of the pellet in suitable buffers without any Ca²⁺ or with rather low Ca²⁺-concentration to reduce the overall Ca²⁺-concentration. Preferably, re-solubilisation in step c1) is accomplished by adding suitable aqueous buffers, which contain chelating agents like EDTA or EGTA to the pellet of the precipitated fusion polypeptide.

In sub-step c2), the re-solubilised fusion polypeptide is then incubated as disclosed above to enable autoproteolysis. The incubation time is preferably 16 to 60 hours, more preferably 16 to 48 hours.

Eventually, step d) of this embodiment comprises the sub-steps of
d1) precipitating the purification domain and cleavage domain by adding Ca²⁺, preferably Ca²⁺-containing buffer, and
d2) isolating the target peptide.

In sub-step d1), the remaining fragment of the purification domain and cleavage domain is precipitated by adding Ca²⁺, preferably Ca²⁺-containing buffer, as described above.

In step d2), the precipitated fragment of the purification domain and cleavage domain is pelletized by centrifugation and, thus, separated from the target peptide or protein, which remains in the supernatant.

The target peptide or protein may optionally be purified further and ultimately isolated by additional measures such as heat treatment, salting out, isoelectric precipitation, trichloroacetic acid (TCA) precipitation, ammonium sulphate precipitation, solvent precipitation and mixtures thereof.

In another preferred embodiment of the first method for preparing a target peptide or protein according to the invention, where a fusion polypeptide with a cleavage domain comprising a nickel-assisted cleavable amino acid sequence X^{a}SX^{b}X^{c}X^{d}X^{e}X^{f}X^{g} as disclosed above is used, step a) includes the sub-steps of
a1) providing a fusion polypeptide comprising a cleavage domain, which comprises a nickel-assisted cleavable amino acid sequence X^{a}SX^{b}X^{c}X^{d}X^{e}X^{f}X^{g} as described above, by recombinant expression in soluble form or in inclusion bodies (IBs),
and, when provided in inclusion bodies,
a2) solubilising the inclusion bodies, and
a3) refolding the fusion polypeptide.

In sub-step a1) of this embodiment, the fusion polypeptide may be provided in soluble form or in the form of insoluble IBs. Recombinant expression in soluble form comprises secretory expression, cytoplasmic expression and periplasmic expression of the fusion polypeptide. As cleavage is exclusively initiated in the presence of nickel, no or at least no significant premature cleavage occurs when expressing the fusion polypeptide in soluble form. The soluble fusion polypeptide may be obtained from the culture medium in case of secretory expression or by lysis of the host cells after culturing in case of cytoplasmic expression and periplasmic expression. In the latter case, it may be necessary to further isolate the periplasmic protein fraction by measures known in the art, such as detergents, alcohols, salts and sugars. When the fusion polypeptide is provided in IBs in sub-step a1), the IBs are preferably obtained by lysis of the host cells after culturing, pelletizing via centrifugation, re-suspending the IB pellet in suitable aqueous washing buffers (e.g. containing 1% Triton or 1 M NaCl) or ddH₂O and another pelletizing via centrifugation. More preferably, re-suspending the IB pellet in suitable aqueous washing buffers (e.g. containing 1% Triton or 1 M NaCl) or ddH₂O and subsequent pelletizing via centrifugation is repeated one or more times for removing other cellular components, wherein the applied washing buffers may vary.

In sub-step a2), the IBs, preferably in the form of a pellet after washing as outlined above, are solubilised by methods known in the art. Preferably, the IBs are solubilized in suitable chaotropic buffers, i.e. aqueous buffers having high concentrations of chaotropic agents such as urea, thiourea, guanidinium thiocyanate and guanidinium chloride/guanidinium hydrochloride or mixtures thereof, optionally including reducing agents to abolish false formed disulfide bonds in the fusion polypeptide. A preferred example of a suitable chaotropic buffer is an aqueous buffer containing 6 M guanidinium chloride/guanidinium hydrochloride and 100 mM HEPES at a pH of 8.2.

In sub-step a3), the solubilized fusion polypeptide is refolded by methods known in the art. Specifically, when the fusion polypeptide is solubilized in suitable chaotropic buffers in sub-step a2), the solubilized fusion polypeptide is preferably refolded by lowering the concentration of the chaotropic agents by dilution. Preferably, suitable kosmotropic buffers are used for dilution, such as aqueous buffers containing no or low concentrations of chaotropic agents. The temperature in sub-step a3) is preferably between -10 and 40°C, more preferably 0 to 30°C, in particular 10 to 25°C. The pH may broadly vary in sub-step a3), it may be from 3 to 12, from 4 to 11, from 5 to 10 or from 6 to 9. A preferred example of a kosmotropic buffer suitable for the refolding of the fusion protein in this embodiment is an aqueous buffer containing 1 M Tris-HCI, 250 mM sucrose, and 5 to 20 mM monothioglycerol at a pH of 8.0 to 8.5. Optionally, arginine may be added to the suitable kosmotropic buffers in concentrations known in the art (e.g. up to 1.0 M, preferably 0.4-0.6 M) for assisting the refolding of the fusion polypeptide.

In step b) of this embodiment, the fusion polypeptide is precipitated as disclosed above by adding Ca²⁺, preferably Ca²⁺-containing buffer. The precipitated fusion polypeptide is then pelletized by centrifugation.

In this embodiment, the cleavage step c) comprises the sub-steps of
c1) re-suspending the precipitated fusion polypeptide obtained in step b) in Ni²⁺-containing buffer, and
c2) incubating the re-suspended fusion polypeptide for 10 min to 40 hours at 15 to 85°C for cleaving the target peptide from the fusion polypeptide by nickel-induced cleavage of the amino acid sequence X^{a}SX^{b}X^{c}X^{d}X^{e}X^{f}X^{g};

Re-suspension in sub-step c1) may be accomplished as disclosed above, preferably by adding Ni²⁺-containing buffer to the pellet of the precipitated fusion polypeptide obtained in step b). Thereby, selective and efficient cleavage of the fusion polypeptide is initiated.

In sub-step c2), the re-suspended fusion polypeptide is then incubated as disclosed above for further cleavage. The incubation time is preferably from 5 to 35 hours, more preferably from 5 to 24 hours and most preferably from 6 to 16 hours. Further, the incubation temperature preferably ranges from 30 to 60°C and more preferably from 40 to 55°C.

Eventually, step d) of this embodiment comprises the sub-steps of
d1) precipitating the purification domain and cleavage domain by adding Ca²⁺, preferably Ca²⁺-containing buffer, and
d2) isolating the target peptide.

In sub-step d1), the remaining fragment of the purification domain and cleavage domain is precipitated by adding Ca²⁺, preferably Ca²⁺-containing buffer, as described above.

In step d2), the precipitated fragment of the purification domain and cleavage domain is pelletized by centrifugation and, thus, separated from the target peptide or protein, which remains in the supernatant.

The target peptide or protein may optionally be purified further and ultimately isolated by additional measures such as heat treatment, salting out, isoelectric precipitation, trichloroacetic acid (TCA) precipitation, ammonium sulphate precipitation, solvent precipitation and mixtures thereof.

A second method for preparing a target peptide or protein according to the invention comprises the steps of
a) providing a fusion polypeptide according to the invention by recombinant expression, wherein the cleavage domain comprises the amino acid sequence X^{a}SX^{b}X^{c}X^{d}X^{e}X^{f}X^{g} as defined above;
b) cleaving the target peptide from the fusion polypeptide;
c) precipitation of the purification domain and cleavage domain by adding Ca²⁺; and
d) recovering the target peptide.

In this alternative method, the fusion polypeptide is first cleaved for releasing the target peptide and, subsequently, the remaining purification domain and cleavage domain fragment is precipitated for purification and isolation.

In step a) of the second method, a fusion polypeptide according to the invention is provided by recombinant expression. This comprises providing a host cell expressing the fusion polypeptide. Such a host cell is obtainable by introducing a nucleic acid molecule including a sequence encoding the fusion polypeptide, preferably in the form of a vector, into the cell by known methods, such as transfection or transformation.

The host cell is cultured in a suitable culture medium, e.g. in a culture medium conventionally used for the respective cell type. Culturing is conducted under conditions, wherein expression of the fusion polypeptide is enabled. For instance, an inducible promoter, such as an auto-inducible, chemically or thermally inducible promoter, may be used to control the expression of the fusion polypeptide.

The fusion polypeptide used in this alternative method comprises a cleavage domain, which contains the nickel-assisted cleavable amino acid sequence X^{a}SX^{b}X^{c}X^{d}X^{e}X^{f}X^{g} as defined above.

Preferably, the fusion polypeptide is expressed in insoluble inclusion bodies (IBs) in step a). The IBs are preferably obtained by lysis of the host cells after culturing, pelletizing via centrifugation, re-suspending the IB pellet in suitable aqueous washing buffers (e.g. containing 1% Triton or 1 M NaCl) or ddH₂O and another pelletizing via centrifugation. More preferably, re-suspending the IB pellet in suitable aqueous washing buffers (e.g. containing 1% Triton or 1 M NaCl) or ddH₂O and subsequent pelletizing via centrifugation is repeated one or more times for removing other cellular components, wherein the applied washing buffers may vary.

In step b) of the second method, the target peptide or protein is cleaved from the fusion polypeptide. Cleavage is chemically induced in the presence of nickel. As disclosed above for the fusion polypeptide of the present invention, the amino acid sequence X^{a}SX^{b}X^{c}X^{d}X^{e}X^{f}X^{g} enables the highly efficient and selective chemically induced cleavage of the sequence before the conserved serine residue in the presence of Ni²⁺.

As indicated previously, the nickel-assisted cleavable amino acid sequence X^{a}SX^{b}X^{c}X^{d}X^{e}X^{f}X^{g} does not require any specific active conformation or the like but cleavage is chemically induced in the presence of Ni²⁺. Accordingly, when the fusion polypeptide is provided in step a) as insoluble IBs, cleavage is preferably accomplished by solubilising the IBs in Ni²⁺-containing chaotropic buffer. That is, even under chaotropic conditions, robust, highly selective and efficient nickel-assisted cleavage of the fusion polypeptide is achieved in this method of the present invention. Put another way, in step b) of the second method, the fusion polypeptide in the form of insoluble IBs is preferably solubilised Ni²⁺-containing chaotropic buffer and, simultaneously, cleavage of the target peptide or protein off from the fusion polypeptide is induced due to the presence of nickel.

It is further preferred that the Ni²⁺-containing chaotropic buffer exhibits a Ni²⁺-concentration of from 0.1 to 25 mM, more preferably from 0.5 to 15 mM, even more preferably from 0.5 to 10 mM, and most preferably from 1 to 5 mM, in particular 3 mM. In addition, it is preferred that the Ni²⁺-containing chaotropic buffer exhibits high concentrations of chaotropic agents such as urea, thiourea, guanidinium thiocyanate and guanidinium chloride/guanidinium hydrochloride or mixtures thereof, and, optionally includes reducing agents to abolish false formed disulfide bonds in the fusion polypeptide. A preferred example of a suitable Ni²⁺-containing chaotropic buffer is an aqueous buffer containing 3 mM NiSO₄, 6 M guanidinium hydrochloride, and 100 mM HEPES at a pH of 8.2.

After solubilisation in Ni²⁺-containing chaotropic buffer, the fusion polypeptide is preferably incubated for further cleavage. The incubation time for cleaving the target peptide domain from the fusion polypeptide by the nickel-assisted cleavage preferably ranges from 10 min to 40 hours, more preferably from 5 to 35 hours, even more preferably from 5 to 24 hours and most preferably from 6 to 16 hours. Preferably, quantitative cleavage is obtained by nickel-assisted cleavage after the incubation time. The incubation temperature is not particularly limited in this step and may range from 15 to 85°C, preferably from 30 to 60°C and more preferably from 40 to 55°C.

As indicated previously, it is another advantage of using a cleavage domain comprising a nickel-assisted cleavable amino acid sequence X^{a}SX^{b}X^{c}X^{d}X^{e}X^{f}X^{g} that the cleavage is accomplished rather quick and at low costs. Still another advantage is that peptides or proteins of interest having an authentic C-terminus may be prepared. Particularly the use of the preferred the amino acid sequence SRHWAP (SEQ ID No: 6) allows for the preparation of peptides or proteins of interest having an authentic C-terminus as the cleavage generally occurs before the conserved serine residue. Hence, provided that the C-terminus of the target peptide domain is directly fused to the N-terminus of the amino acid sequence SRHWAP, which is especially preferred, an authentic C-terminus of the target peptide domain is obtained, i.e. no amino acid residues of the cleavage domain or of any linker remain with the target peptide domain. An authentic N-terminus of the target peptide domain may simultaneously be obtained by an appropriate selection of the expression system for the fusion polypeptide, particularly of the host cell.

In step c) of the second method, the purification domain and cleavage domain are precipitated by adding Ca²⁺. As used in this context, the term *"adding Ca²⁺"* encompasses contacting the remaining fragment of the purification domain and cleavage domain with Ca²⁺. This causes the calcium-responsive purification domain to form a β-roll structure leading to precipitation of the fragment whereas the cleaved-off peptide or protein of interest remains in solution.

Preferably, after solubilisation and incubation in Ni²⁺-containing chaotropic buffer for cleavage in step b), the reaction mixture including the fragment of the purification domain and cleavage domain as well as the target peptide or protein is first refolded by methods known in the art, more preferably by lowering the concentration of the chaotropic agents in the Ni²⁺-containing chaotropic buffer by dilution. Preferably, suitable kosmotropic buffers are used for dilution, such as aqueous buffers containing no or low concentrations of chaotropic agents. An example of a preferred kosmotropic buffer for refolding is an aqueous buffer containing 1 M Tris-HCI, 250 mM sucrose, and 5 to 20 mM monothioglycerol at a pH of 8.0 to 8.5. After refolding, Ca²⁺, preferably Ca²⁺-containing buffer, such as aqueous buffer containing CaCl₂, CaCO₃, Ca(OH)₂, Ca(NO₃)₂ or CaSO₄, is added for precipitation of the refolded fragment of the purification domain and cleavage domain.

More preferably, after solubilisation and incubation in Ni²⁺-containing chaotropic buffer for cleavage in step b), the reaction mixture including the fragment of the purification domain and cleavage domain as well as the target peptide or protein is refolded in step c) by lowering the concentration of the chaotropic agents in the Ni²⁺-containing chaotropic buffer by dilution using Ca²⁺-containing kosmotropic buffers, such as aqueous buffers containing CaCl₂, CaCO₃, Ca(OH)₂, Ca(NO₃)₂ or CaSO₄. Thereby, refolding of the reaction mixture and highly specific and efficient precipitation of the fragment of the purification domain and cleavage domain are combined in one step.

Further, it is preferred that the Ca²⁺-concentration in step c) ranges from 25 to 500 mM, more preferably from 25 to 300 mM, even more preferably from 50 to 250 mM, and most preferably from 75 to 200 mM. Accordingly, it is preferred that the Ca²⁺-containing buffer or the Ca²⁺-containing kosmotropic buffer each exhibit a Ca²⁺-concentration of from 25 to 500 mM, more preferably of from 50 to 300 mM, even more preferably of from 75 to 250 mM, and most preferably of rom 75 to 200 mM. The further composition of the Ca²⁺-containing buffer or of the Ca²⁺-containing kosmotropic buffer is not particularly limited as long as it contains a sufficient amount of Ca²⁺ for precipitating the fragment of the purification domain and cleavage domain as indicated above. An example of a preferred Ca²⁺-containing kosmotropic buffer for refolding and precipitating the fragment of the purification domain and cleavage domain in step c) is an aqueous buffer containing 100 mM CaCl₂, 122 mM citric acid, 38 mM Na₂HPO₄ at a pH of 4.0. Another preferred Ca²⁺-containing buffer for refolding and precipitating the fragment of the purification domain and cleavage domain in step c) is an aqueous buffer containing 100 mM CaCl₂, 1 M Tris base, 250 mM sucrose, 10 mM monothioglycerol, pH 8.2.

The incubation time of the precipitation step c) preferably ranges from 1 min to 16 hours, more preferably from 1 min to 8 hours, even more preferably from 1 to 90 min, still more preferably from 1 to 60 min, and most preferably from 5 to 45 min. Accordingly, precipitation step c) is not time consuming but rather quick and cost efficient as no chromatographic purification is required. Accordingly, step c) of the second method according to the invention allows for highly selective and efficient precipitation and, ultimately, removal of the fragment of the purification domain and cleavage domain.

Preferably, the fragment of the purification domain and cleavage domain precipitated in step c) is pelletized by centrifugation whereas the target peptide or protein remains in the solution in the supernatant.

In step d) of the second method according to the invention, the target peptide or protein is recovered.

For this purpose, step d) preferably comprises conventional measures known in the art, such as heat treatment, salting out, isoelectric precipitation, trichloroacetic acid (TCA) precipitation, ammonium sulphate precipitation, solvent precipitation and mixtures thereof. A chromatographic purification step, however, is not necessary for the recovery of the target peptide or protein in high yield and high purity, which also renders the second method for the preparation thereof according the present invention less time consuming and less expensive.

In a preferred embodiment of the second method for preparing a target peptide or protein according to the invention, step a) includes providing the fusion polypeptide in inclusion bodies (IBs). The IBs are preferably obtained and washed as disclosed above for the second method of the invention.

In said preferred embodiment, step b) comprises the sub-steps of
b1) solubilising the inclusion bodies in Ni²⁺-containing buffer, and
b2) incubating the solubilized fusion polypeptide for 10 min to 40 hours at 15 to 85°C for cleaving the target peptide from the fusion polypeptide by nickel-induced cleavage of the amino acid sequence X^{a}SX^{b}X^{c}X^{d}X^{e}X^{f}X^{g}.

In sub-step b1), the fusion polypeptide is preferably solubilised in Ni²⁺-containing chaotropic buffer and, simultaneously, cleavage of the target peptide or protein off from the fusion polypeptide is induced due to the presence of nickel as disclosed above.

In sub-step b2), the incubation time for further cleavage is preferably 5 to 35 hours, more preferably 5 to 24 hours, and most preferably 6 to 16 hours. The incubation temperature is preferably 30 to 60°C, and more preferably 40 to 55°C.

Step c) comprises refolding the purification domain and cleavage domain as well as the target peptide obtained in step b2) in Ca²⁺-containing buffer, thereby precipitating the purification domain and cleavage domain.

In other words, refolding of the reaction mixture and highly specific and efficient precipitation of the fragment of the purification domain and cleavage domain are combined in one step in step c) of this preferred embodiment. The incubation time in step c) is as outlined above. Preferably, the fragment of the purification domain and cleavage domain precipitated in step c) is pelletized by centrifugation whereas the target peptide or protein remains in the solution in the supernatant.

Eventually, step d) of the preferred embodiment of the second method according to the invention comprises isolating the target peptide, preferably by trichloroacetic acid (TCA) precipitation or by HCI precipitation, or by solvent precipitation.

### Benefits of the invention

The present invention is based on a fusion polypeptide that enables highly efficient purification together with highly specific and efficient protein cleavage for releasing the peptide or protein of interest, preferably having authentic termini.

Accordingly, the methods of the invention for the preparation of a peptide or protein of interest using said fusion polypeptide enable a highly efficient purification by simple and robust operation together with a highly specific and efficient protein cleavage for releasing the peptide or protein of interest. In particular, there is no need for one or more time consuming and costly chromatographic purification steps. Likewise, the costly use of an external protease is avoided. As a result, a peptide or protein of interest, preferably having authentic termini, can be provided in industrial scale at high yields and, simultaneously, at low overall costs.

### Examples

The present invention is described in more detail by the following Examples.

### Example 1

### Expression of (GGAGNDTLY)₇-Δ1-16 N^{Pro} EDDIE-Lfc and isolation of IBs

In Example 1, a fusion polypeptide containing, from the N-terminus to the C-terminus, seven tandem repeats of a nonapeptide having the sequence GGAGNDTLY as the purification domain, the autoprotease Δ1-16 CSFV N^{Pro} EDDIE derivative as the cleavage domain and lactoferricin (Lfc) as a model target peptide domain ((GGAGNDTLY)₇-Δ1-16 N^{Pro} EDDIE-Lfc) was prepared by recombinant expression in inclusion bodies. The model target peptide Lfc carried a C-terminal hexahistidine tag.

Specifically, a BL21-based *E. coli* strain carrying a pET-vector having kanamycin resistance and a gene sequence (SEQ ID No: 8) encoding the above fusion polypeptide (GGAGNDTLY)₇₋Δ1-16 N^{Pro} EDDIE-Lfc (SEQ ID No: 9) was inoculated with kanamycin and chloramphenicol in 1 L LB medium. At an OD₆₀₀ of 0.5, expression was induced by addition of 0.135 mM IPTG. After 16 hours of expression, the culture was centrifuged at 3500 x g for 30 min, the harvested host cells were washed with 0.1 M NaCl, and centrifuged again at 5000 x g for 15 min. Lysis buffer (50 mM HEPES, pH 8.2) was added to the cell pellet in an amount of 16 ml/g pellet, and the cells were disrupted at 30% intensity (165 W) in a Branson ultrasonic homogenizer and the lysed cells were centrifuged again at 5000 x g for 10 minutes. For washing, the obtained pellet was twice resuspended in wash buffer 1 (1% Triton) by further sonification followed by centrifugation at 5000 x g for 15 minutes, once re-suspended in wash buffer 2 (1 M NaCl) by further sonification followed by centrifugation at 5000 x g for 15 minutes, and once re-suspended in ddH₂O by further sonification and again centrifuged at 5000 x g for 15 minutes. The obtained pellet of the IBs was stored at -30°C until further use.

### Example 2

### Expression of Lfc-SRHWAP-(GGAGNDTLY)₁₇-CAP and isolation of IBs

In Example 2, a fusion polypeptide containing, from the N-terminus to the C-terminus, lactoferricin (Lfc) as a model target peptide domain, a nickel-assisted cleavable amino acid sequence SRHWAP as the cleavage domain, and seventeen tandem repeats of a nonapeptide having the sequence GGAGNDTLY followed by a capping sequence as the purification domain (Lfc-SRHWAP-(GGAGNDTLY)₁₇-CAP), was prepared by recombinant expression in inclusion bodies. The model target peptide Lfc carried an N-terminal hexahistidine tag while the purification domain carried a hexahistidine tag followed by an ssTorA tag at the C-terminus.

Specifically, a BL21-based *E. coli* strain carrying a pET-vector having kanamycin resistance and a gene sequence (SEQ ID No: 10) encoding the fusion polypeptide Lfc-SRHWAP-(GGAGNDTLY)₁₇-CAP (SEQ ID No: 11) was inoculated with kanamycin and chloramphenicol in 1 L LB medium. At an OD₆₀₀ of 0.5, expression was induced by addition of 0.135 mM IPTG. After 16 hours of expression, the culture was centrifuged at 3500 x g for 30 min, the harvested host cells were washed with 0.1 M NaCl, and centrifuged again at 5000 x g for 15 min. Lysis buffer (50 mM HEPES, pH 8.2) was added to the cell pellet in an amount of 16 ml/g pellet, and the cells were disrupted at 30% intensity (165 W) in a Branson ultrasonic homogenizer and the lysed cells were centrifuged again at 5000 x g for 10 minutes. For washing, the obtained pellet was twice resuspended in wash buffer 1 (1% Triton) by further sonification followed by centrifugation at 5000 x g for 15 minutes, once re-suspended in wash buffer 2 (1 M NaCl) by further sonification followed by centrifugation at 5000 x g for 15 minutes, and once re-suspended in ddH₂O by further sonification and again centrifuged at 5000 x g for 15 minutes. The obtained pellet of the IBs was stored at -30°C until further use.

### Example 3

### Expression of FGF2-SRHWAP-(GGAGNDTLY)₁₇-CAP and isolation of IBs

In Example 3, a fusion polypeptide containing, from the N-terminus to the C-terminus, basic fibroblast growth factor (FGF2) as a model target peptide domain, a nickel-assisted cleavable amino acid sequence SRHWAP as the cleavage domain, and seventeen tandem repeats of a nonapeptide having the sequence GGAGNDTLY followed by a capping sequence as the purification domain (Lfc-SRHWAP-(GGAGNDTLY)₁₇-CAP), was prepared by recombinant expression in inclusion bodies. The model target peptide FGF2 carried an N-terminal hexahistidine tag while the purification domain carried a C-terminal hexahistidine tag.

Specifically, a BL21-based *E. coli* strain carrying a pET-vector having kanamycin resistance and a gene sequence (SEQ ID No: 12) encoding the fusion polypeptide FGF2-SRHWAP-(GGAGNDTLY)₁₇-CAP (SEQ ID NO: 13) was inoculated with kanamycin and chloramphenicol in 1 L LB medium. At an OD₆₀₀ of 0.5, expression was induced by addition of 0.135 mM IPTG. After 16 hours of expression, the culture was centrifuged at 3500 x g for 30 min, the harvested host cells were washed with 0.1 M NaCl, and centrifuged again at 5000 x g for 15 min. Lysis buffer (50 mM HEPES, pH 8.2) was added to the cell pellet in an amount of 16 ml/g pellet, and the cells were disrupted at 30% intensity (165 W) in a Branson ultrasonic homogenizer and the lysed cells were centrifuged again at 5000 x g for 10 minutes. For washing, the obtained pellet was twice resuspended in wash buffer 1 (1% Triton) by further sonification followed by centrifugation at 5000 x g for 15 minutes, once re-suspended in wash buffer 2 (1 M NaCl) by further sonification followed by centrifugation at 5000 x g for 15 minutes, and once re-suspended in ddH₂O by further sonification and again centrifuged at 5000 x g for 15 minutes . The obtained pellet of the IBs was stored at -30°C until further use.

### Example 4

### Preparation of Lfc using (GGAGNDTLY)₇-Δ1-16 N^{Pro} EDDIE-Lfc

The pellet of the IBs obtained in Example 1 was solubilized in chaotropic buffer (6 M guanidinium hydrochloride, 100 mM HEPES, pH 8.2) and adjusted to a protein concentration of 5 mg/ml. The solubilized fusion polypeptide (GGAGNDTLY)₇₋Δ1-16 N^{Pro} EDDIE-Lfc was refolded by 20-fold dilution in a kosmotropic buffer (1 M Tris-HCI, 250 mM sucrose, 2 mM EDTA, 10 mM monothioglycerol, pH 8.2) at 18°C. After incubation at 18°C for 30 min, the fusion polypeptide was precipitated by adding Ca²⁺-containing buffer (2 M CaCl₂) in an amount such that a final concentration of CaCl₂ of 150 mM was obtained and incubation for 5 min at 18°C. The precipitated fusion polypeptide was then pelletized by centrifugation for 2 min at 16.000 x g. The obtained pellet was once re-suspended in washing buffer (1 M Tris-HCI, 250 mM sucrose, 2 mM EDTA, 25 mM CaCl₂, and 10 mM monothioglycerol, pH 8.2) and again pelletized by centrifugation for 2 min at 16.000 x g. After washing, the precipitated fusion polypeptide was re-solubilized in a volume of re-solubilisation buffer (50 mM EDTA, 1 M Tris-HCI, 250 mM sucrose, and 10 mM monothioglycerol, pH 8.2) equal to that after refolding and incubated for 48 hours at 18 °C to allow for cleavage of Lfc from the fusion polypeptide by autoproteolysis. Thereafter, the fragment (GGAGNDTLY)₇₋Δ1-16 N^{Pro} EDDIE was precipitated by adding Ca²⁺-containing buffer (2 M CaCl₂) in an amount such that a final concentration of CaCl₂ of 200 mM was obtained. After incubation for 60 min at 18°C, the precipitated fragment (GGAGNDTLY)₇₋Δ1-16 N^{Pro} EDDIE was separated from the target protein Lfc by centrifugation at 16000 x g for 5 min.

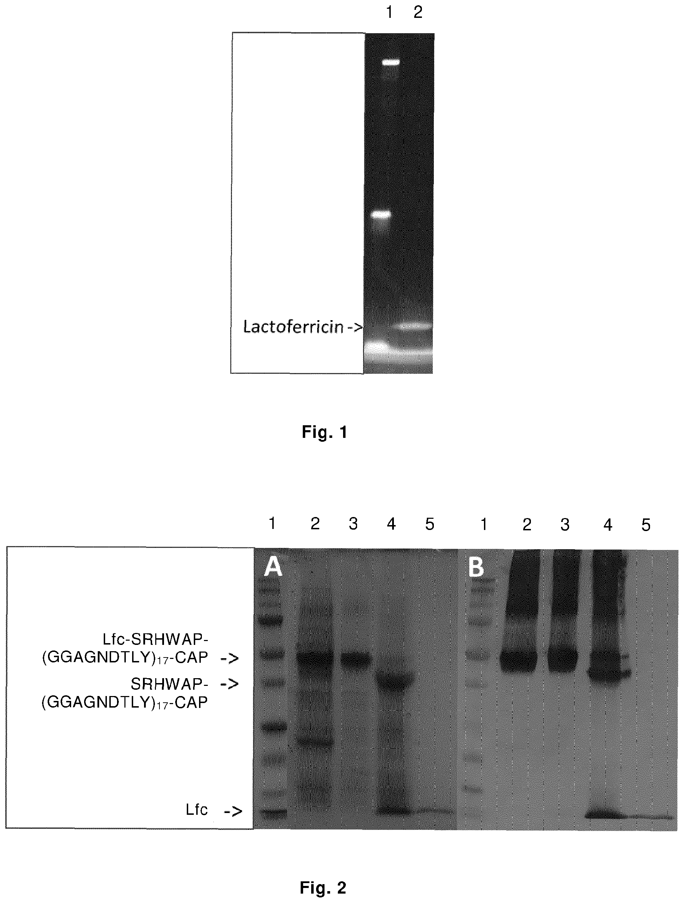

The obtained purity of the target peptide Lfc was analysed via SDS-PAGE. In Figure 1, the corresponding SDS-PAGE gel (BioRad Pre-Stained Gel) under UV is shown. Lane 1 contains the molecular weight standard (BioRad Precision Plus Protein^{™} Dual Xtra) and lane 2 the supernatant after separation of the precipitated fragment (GGAGNDTLY)₇₋Δ1-16 N^{pro} EDDIE from the target protein Lfc by centrifugation. As can be readily derived, the target protein Lfc is obtained in high purity.

### Example 5

### Preparation of Lfc using Lfc-SRHWAP-(GGAGNDTLY)₁₇-CAP

The pellet of the IBs obtained in Example 2 was solubilized in Ni²⁺-containing chaotropic buffer (6 M guanidinium hydrochloride, 100 mM HEPES, 3 mM NiSO₄, pH 8.2) and adjusted to a concentration of 5 mg/ml. Thereby, nickel-assisted cleavage of the solubilized fusion polypeptide Lfc-SRHWAP-(GGAGNDTLY)₁₇-CAP was induced. For further cleavage, the solution was incubated for 20 hours at 50°C. Thereafter, the protein in the reaction mixture was refolded by 20-fold dilution in a Ca²⁺-containing kosmotropic buffer (100 mM CaCl₂, 122 mM citric acid, 38 mM Na₂HPO₄, pH 4.0) at 18°C leading to precipitation of the fragment SRHWAP-(GGAGNDTLY)₁₇-CAP. The precipitated fragment was pelletized by centrifugation at 5000 x g for 40 min. The target protein Lfc in the supernatant was precipitated by adding 0.015% deoxycholate and 10% TCA and incubation at -30°C for 15 minutes. The precipitated target peptide was then centrifuged at 21000 x g for 10 minutes. The resulting pellet was washed twice by resuspending it in -30 °C cold acetone followed by centrifugation at 16000 x g for 10 min and then re-suspended in aqueous buffer (122 mM citric acid, 38 mM Na₂HPO₄, 100 mM CaCl₂, pH 4).

The progress in the preparation of the target peptide Lfc and its final purity were analysed via SDS-PAGE and western-blotted using a monoclonal mouse antipolyhistidine antibody (H1029 from Sigma-Aldrich, dilution 1:3000) together with a secondary rabbit anti-mouse IgG2a antibody conjugated with alkaline phosphatase (610-4541 from Rockland Inc., dilution 1:1500). In Figure 2 A, the corresponding SDS-PAGE gel (stained with Comassie brilliant blue) is shown while the corresponding western blot is illustrated in Figure 2 B (detection via alkaline phosphatase and BCIP and NBT as substrate). Lane 1 each contains the molecular weight standard (BioRad Precision Plus Protein^{™} Dual Xtra) and lane 2 each contains the complete protein extract obtained by lysis of the cultured host cells in Example 3. Lane 3 each contains the washed IBs obtained in Example 3. Lane 4 contains the reaction mixture after solubilisation in Ni²⁺-containing chaotropic buffer and subsequent incubation. As can be derived from lane 4 in Figures 2 A and 2 B, the target protein LFc is almost quantitatively cleaved off from the fusion polypeptide Lfc-SRHWAP-(GGAGNDTLY)₁₇-CAP. Lane 5 each contains the target protein LFc after refolding in Ca²⁺-containing kosmotropic buffer and subsequent recovery steps as indicated above. It is apparent, that the target protein LFc is obtained in excellent purity.

### Example 6

### Preparation of Lfc using Lfc-SRHWAP-(GGAGNDTLY)₁₇-CAP

The pellet of the IBs obtained in Example 2 was solubilized in Ni²⁺-containing chaotropic buffer (6 M guanidinium hydrochloride, 100 mM HEPES, 3 mM NiSO₄, pH 8.2) and adjusted to a concentration of 5 mg/ml. Thereby, nickel-assisted cleavage of the solubilized fusion polypeptide Lfc-SRHWAP-(GGAGNDTLY)₁₇-CAP was induced. For further cleavage, the solution was incubated for 20 hours at 50°C. Thereafter, the protein in the reaction mixture was refolded by 20-fold dilution at 18°C in a Ca²⁺-containing kosmotropic buffer (100 mM CaCl₂, 122 mM citric acid, 38 mM Na₂HPO₄, pH 4.0) and incubated for 30 min at 50°C, leading to precipitation of the fragment SRHWAP-(GGAGNDTLY)₁₇-CAP. The precipitated fragment was pelletized by centrifugation at 21000 x g for 5 min. The target protein Lfc in the supernatant was precipitated by adding 10% TCA and isolated via centrifugation at 16000 x g for 2 min.

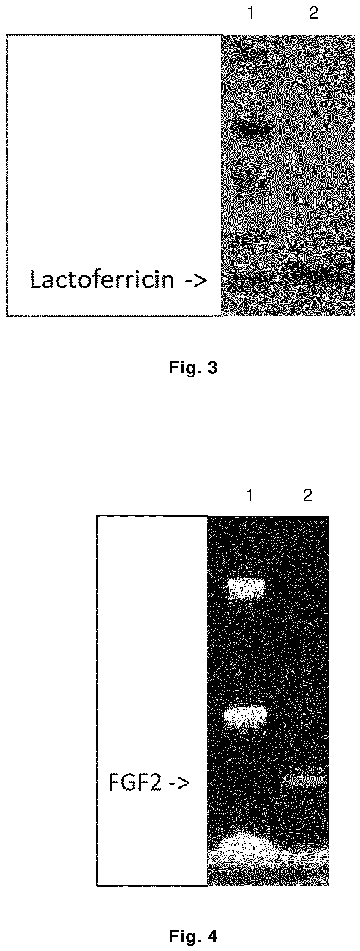

The obtained purity of the target peptide Lfc was analysed via SDS-PAGE. In Figure 3, the corresponding SDS-PAGE gel (stained with Coomassie brilliant blue) is shown. Lane 1 contains the molecular weight standard (BioRad Precision Plus Protein^{™} Dual Xtra) and lane 2 the isolated target protein Lfc. As can be readily derived, the target protein Lfc is obtained in excellent purity.

### Example 7

### Preparation of FGF2 using FGF2-SRHWAP-(GGAGNDTLY) ₁₇-CAP

The pellet of the IBs obtained in Example 3 was solubilized in Ni²⁺-containing chaotropic buffer (6 M guanidinium hydrochloride, 100 mM HEPES, 3 mM NiSO₄, pH 8.2) and adjusted to a concentration of 5 mg/ml. Thereby, nickel-assisted cleavage of the solubilized fusion polypeptide Lfc-SRHWAP-(GGAGNDTLY)₁₇-CAP was induced. For further cleavage, the solution was incubated for 20 hours at 50°C. Thereafter, the protein in the reaction mixture was refolded at 18°C by 20-fold dilution in a kosmotropic buffer (1 M Tris-HCI, 250 mM sucrose, 10 mM monothoglycerol, pH 8.0) and Ca²⁺-containing buffer (2 M CaCl₂) was added in an amount such that a final concentration of 100 mM CaCl₂ was obtained for precipitation of the fragment SRHWAP-(GGAGNDTLY)₁₇-CAP. After further incubation for 30 min at room temperature, the precipitated fragment was pelletized by centrifugation at 21000 x g for 5 min. The target protein FGF2 in the supernatant was precipitated by adding concentrated HCI (37%) to a final concentration of 1.85 % and incubation for 30 min at room temperature. The target protein FGF2 was then isolated via centrifugation at 16000 x g for 2 min.

The obtained purity of the target peptide FGF2 was analysed via SDS-PAGE. In Figure 4, the corresponding SDS-PAGE gel (BioRad Pre-Stained Gel) under UV is shown. Lane 1 contains the molecular weight standard (BioRad Precision Plus Protein^{™} Dual Xtra) and lane 2 the isolated target protein FGF2. As can be readily derived, the target protein FGF 2 is obtained in very high purity.

### Example 8

### Preparation of Lfc using Ltc-SRHWAP-(GGAGNDTL Y)₁₇-CAP

The pellet of the IBs obtained in Example 2 was solubilized in chaotropic buffer (6 M guanidinium hydrochloride, 100 mM HEPES, pH 8.2) and adjusted to a protein concentration of 5 mg/ml. The solubilized fusion polypeptide Lfc-SRHWAP-(GGAGNDTLY)₁₇-CAP was refolded by 20-fold dilution in a kosmotropic buffer (1 M Tris-HCI, 250 mM sucrose, 10 mM monothioglycerol, pH 8.0) at 18°C. After incubation at 18°C for 15 min, the fusion polypeptide was precipitated by adding Ca²⁺-containing buffer (2 M CaCl₂) in an amount such that a final concentration of 90 mM CaCl₂ was obtained and further incubation for 5 min at 18°C. The precipitated fusion polypeptide was then pelletized by centrifugation for 2 min at 16000 x g. The obtained pellet was once re-suspended in washing buffer (100 mM HEPES, 25 mM CaCl₂, 10 mM monothioglycerol, pH 8.2) and again pelletized by centrifugation for 2 min at 16.000 x g. After washing, the precipitated fusion polypeptide was re-suspended in a volume of Ni²⁺-containing buffer (100 mM HEPES, 3 mM NiSO₄, pH 8.2) equal to that after refolding and incubated for 20 hours at 50 °C to allow for cleavage of the target protein Lfc from the fusion polypeptide by nickel-assisted cleavage. Thereafter, the fragment SRHWAP-(GGAGNDTLY)₁₇-CAP was precipitated by adding Ca²⁺-containing buffer (2 M CaCl₂) in an amount such that a final concentration of 100 mM CaCl₂ was obtained. After incubation for 45 min at 18°C, the precipitated fragment SRHWAP-(GGAGNDTLY)₁₇-CAP was separated from the target protein Lfc by centrifugation at 16000 x g for 5 min.

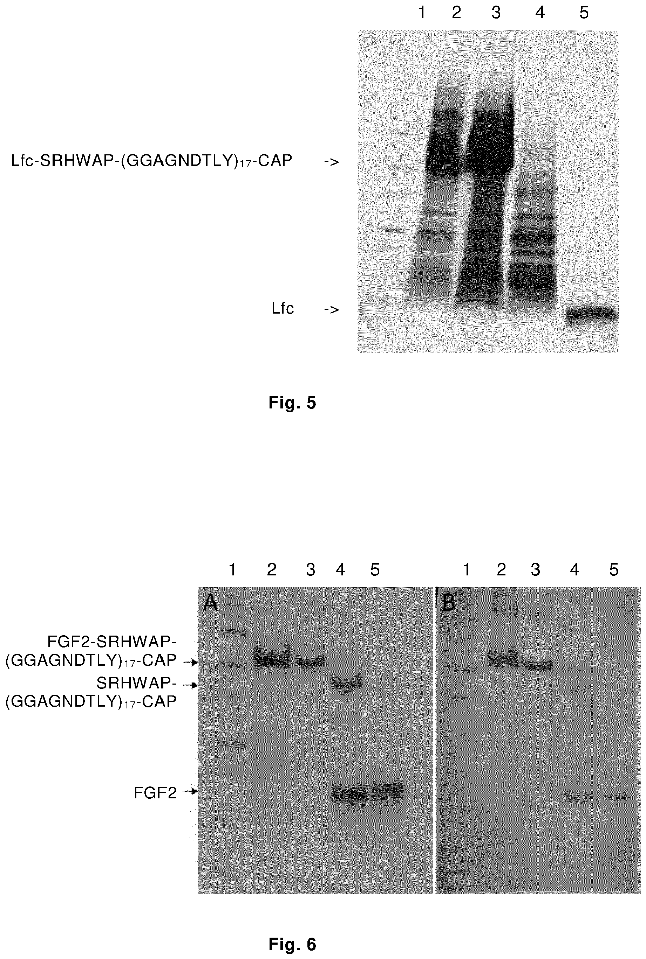

The obtained purity of the target peptide Lfc was analysed via SDS-PAGE. In Figure 5, the corresponding SDS-PAGE gel (stained with Coomassie brilliant blue) is shown. Lane 1 contains the molecular weight standard (BioRad Precision Plus Protein^{™} Dual Xtra) and lane 2 contains the washed IBs obtained in Example 2. Lane 3 contains the reaction mixture after solubilisation and refolding while lane 4 contains the supernatant after precipitation of the fusion polypeptide and subsequent centrifugation. As can be derived therefrom, the fusion polypeptide is quantitatively precipitated in the presence of calcium. Lane 5 contains the supernatant after nickel-assisted cleavage of the target peptide Lfc from the fusion polypeptide, precipitation of the fragment SRHWAP-(GGAGNDTLY)₁₇-CAP in the presence of calcium and subsequent separation of the precipitated fragment SRHWAP-(GGAGNDTLY)₁₇-CAP from the target protein Lfc by centrifugation. It is apparent that the target protein Lfc is obtained in excellent purity.

### Example 9

### Preparation of FGF2 using FGF2-SRHWAP-(GGAGNDTLY)₁₇-CAP

The pellet of the IBs obtained in Example 3 was solubilized in chaotropic buffer (6 M guanidinium hydrochloride, 100 mM HEPES, pH 8.2) and adjusted to a protein concentration of 5 mg/ml. The solubilized fusion polypeptide FGF2-SRHWAP-(GGAGNDTLY)₁₇-CAP was refolded by 20-fold dilution in a kosmotropic buffer (1 M Tris-HCI, 250 mM sucrose, 10 mM monothioglycerol, pH 8.0) at 18°C. After incubation at 18°C for 90 min, the fusion polypeptide was precipitated by adding Ca²⁺-containing buffer (2 M CaCl₂) in an amount such that a final concentration of 100 mM CaCl₂ was obtained and further incubation for 30 min at 18°C. The precipitated fusion polypeptide was then pelletized by centrifugation for 2 min at 16000 x g. The obtained pellet was once re-suspended in washing buffer (100 mM HEPES, 25 mM CaCl₂, 10 mM monothioglycerol, pH 8.2) and again pelletized by centrifugation for 2 min at 16.000 x g. After washing, the precipitated fusion polypeptide was re-suspended in a volume of Ni²⁺-containing buffer (100 mM HEPES, 3 mM NiSO₄, 1,6 M GuHCL, pH 8.2) equal to that after refolding and incubated for 20 hours at 50 °C to allow for cleavage of the target protein FGF2 from the fusion polypeptide by nickel-assisted cleavage. Thereafter, the fragment SRHWAP-(GGAGNDTLY)₁₇-CAP was precipitated by adding Ca²⁺-containing buffer (2 M CaCl₂) to the reaction mixture in an amount such that a final concentration of 100 mM CaCl₂ was obtained. After incubation for 30 min at 18°C, the precipitated fragment SRHWAP-(GGAGNDTLY)₁₇-CAP was separated from the target protein FGF2 by centrifugation at 16000 x g for 5 min.

The progress in the preparation of the target peptide FGF2 and its final purity were analysed via SDS-PAGE and western-blotted using a monoclonal mouse anti-FGF2 antibody FB-8 (MA1-24682 from Invitrogen, dilution 1:2000) in together with a secondary rabbit anti-mouse IgG2a antibody conjugated with alkaline phosphatase (610-4541 from Rockland Inc., dilution 1:1500). In Figure 6 A, the corresponding SDS-PAGE gel (stained with Coomassie brilliant blue) is shown while the corresponding western blot is illustrated in Figure 6 B (detection via alkaline phosphatase and BCIP and NBT as substrate). Lane 1 each contains the molecular weight standard (BioRad Precision Plus Protein^{™} Dual Xtra) while lane 2 each contains the reaction mixture after solubilisation in chaotropic buffer and subsequent refolding. It can be derived that said reaction mixture contains the refolded fusion polypeptide FGF2-SRHWAP-(GGAGNDTLY)₁₇-CAP in high purity. Lane 3 each contains the reaction mixture after precipitation in the presence of calcium and resuspension in washing buffer. It is apparent that highly pure fusion polypeptide is obtained by this procedure. Lane 4 each contains the reaction mixture after resuspension in Ni²⁺-containing buffer and subsequent incubation. It can readily derived that the target protein FGF2 is quantitatively cleaved off from the fusion polypeptide by nickel-assisted cleavage. Lane 5 each contains the supernatant after the second addition of calcium leading to the precipitation of the fragment SRHWAP-(GGAGNDTLY)₁₇-CAP

## Claims

1. A fusion polypeptide, comprising
i) purification domain;
ii) a cleavage domain; and
iii) a target peptide domain;
wherein the purification domain i) comprises 3 to 50 repeats of a nonapeptide, each nonapeptide independently having an amino acid sequence GX²X³X⁴X⁵X⁶X⁷X⁸X⁹, whereby X² may be G, N or D, X³ may be A, S, G, D, E, L or N, X⁴ may be G or A, X⁵ may be N, D, A or S, X⁶ may be D or N, X⁷ may be T, I, V or L, X⁸ may be L, V, I, F or Y, and X⁹ may be Y, I, V, F, T, N, D, K or S; and
wherein the cleavage domain ii) comprises an autoprotease or an amino acid sequence X^{a}SX^{b}X^{c}X^{d}X^{e}X^{f}X^{g}, whereby X^{a} is only optional and may be any amino acid, X^{b} may be H, R, S, N, Y, G, K or Q, X^{c} may be H, S or N, X^{d} may be T, S, L, M, G, N, Q or W, X^{e} is only optional may be S, P, A, T, L, R or D, X^{f} is only optional and may be L, P, E, G, T, A, F or S, and X^{g} is only optional and may be any amino acid.

2. The fusion polypeptide according to claim 1, wherein the purification domain i) comprises 5 to 40, preferably 5 to 25, more preferably 6 to 20, even more preferably 7 to 17, and most preferably 7, 12 or 17 repeats of the nonapeptide.

3. The fusion polypeptide according to claims 1 or 2, wherein X² in each nonapeptide having the an amino acid sequence GX²X³X⁴X⁵X⁶X⁷X⁸X⁹ is G, X⁴ is G, X⁶ is D, and X⁸ is L, I or F, preferably, each nonapeptide has the amino acid sequence GGAGNDTLY.

4. The fusion polypeptide according to any of the preceding claims, wherein the purification domain i) comprises a capping sequence.

5. The fusion polypeptide according to any of the preceding claims, wherein the autoprotease is a viral autoprotease, preferably an autoprotease derived from a virus of the family Flaviviridae, more preferably an autoprotease derived from a pestivirus, and even more preferably an N^{Pro} wildtype autoprotease or an active fragment or an active mutant thereof.

6. The fusion polypeptide according to any of the preceding claims, wherein the autoprotease is an N^{Pro} wildtype autoprotease derived from CSFV or an active mutant thereof, preferably the mutant is CSFV N^{Pro} EDDIE.

7. The fusion polypeptide according to any of the preceding claims, wherein X^{a} in the amino acid sequence x^{a}SX^{b}x^{c}X^{d}x^{e}X^{f}X^{g} is G, X^{b} is H or R, X^{c} is H, X^{d} is W, X^{e} is A, and X^{f} is P, preferably the amino acid sequence X^{a}SX^{b}X^{c}X^{d}X^{e}X^{f}X^{g} is SRHWAP or GSHHW, more preferably, the amino acid sequence X^{a}SX^{b}X^{c}X^{d}X^{e}X^{f}X^{g} is SRHWAP.

8. The fusion polypeptide according to any one of claims 1 to 6, comprising in direction from the N-terminus to the C-Terminus
i) the purification domain, optionally including the capping sequence;
ii) the cleavage domain comprising the autoprotease; and
iii) the target peptide domain.

9. The fusion polypeptide according to any one of claims 1 to 4 and 7, comprising in direction from the N-terminus to the C-Terminus
iii) the target peptide domain;
ii) the cleavage domain comprising the amino acid sequence X^{a}SX^{b}X^{c}X^{d}X^{e}X^{f}X^{g}; and
i) the purification domain, optionally including the capping sequence.

10. A recombinant nucleic acid molecule encoding the fusion polypeptide according to any of the preceding claims, wherein, preferably, the recombinant nucleic acid molecule is arranged in a vector, more preferably, the recombinant nucleic acid molecule is arranged in a plasmid.

11. A host cell comprising the recombinant nucleic acid molecule according to claim 10.

12. A method for preparing a target peptide, comprising the steps of
a) providing a fusion polypeptide according to any one of claims 1 to 9 by recombinant expression;
b) precipitating the fusion polypeptide by adding Ca²⁺, preferably Ca²⁺containing buffer;
c) cleaving the target peptide from the fusion polypeptide; and
d) recovering the target peptide.

13. A method for preparing a target peptide, comprising the steps of
a) providing a fusion polypeptide according to any one of claims 1 to 4, 7 and 9 by recombinant expression, wherein the cleavage domain comprises the amino acid sequence X^{a}SX^{b}X^{c}X^{d}X^{e}X^{f}X^{g};
b) cleaving the target peptide from the fusion polypeptide;
c) precipitation of the purification domain and cleavage domain by adding Ca²⁺, preferably Ca²⁺-containing buffer; and
d) recovering the target peptide.

14. The method according to claim 12 or 13, wherein the Ca²⁺-concentration in the precipitation step ranges from 25 to 500 mM, preferably from 50 to 300 mM, more preferably from 75 to 250 mM, even more preferably from 75 to 200 mM, and wherein the incubation time of the precipitation step ranges from 1 min to 16 hours, preferably from 1 min to 8 hours, more preferably from 1 to 90 min, even more preferably from 1 to 60 min, and most preferably from 5 to 45 min.

15. The method according to claim 12 or 14,
wherein step a) includes the sub-steps of
a1) providing a fusion polypeptide according to any one of claims 1 to 6 and 8 by recombinant expression in inclusion bodies,
a2) solubilising the inclusion bodies, and
a3) refolding the fusion polypeptide;
wherein step c) comprises the sub-steps of
c1) re-solubilising the precipitated fusion polypeptide obtained in step b), and
c2) incubating the re-solubilised fusion polypeptide for 8 to 70 hours, preferably 16 to 60 hours, more preferably 16 to 48 hours, for cleaving the target peptide from the fusion polypeptide by autoproteolysis;
and wherein step d) comprises the sub-steps of
d1) precipitating the purification domain and cleavage domain by adding Ca²⁺, preferably Ca²⁺-containing buffer, and
d2) isolating the target peptide.

16. The method according to claim 12 or 14,
wherein step a) includes the sub-steps of
a1) providing a fusion polypeptide according to any one of claims 1 to 4, 7 and 9 by recombinant expression in soluble form or in inclusion bodies, and, when provided in inclusion bodies,
a2) solubilising the inclusion bodies, and
a3) refolding the fusion polypeptide;
wherein step c) comprises the sub-steps of
c1) resuspending the precipitated fusion polypeptide obtained in step b) in Ni²⁺-containing buffer, and
c2) incubating the resuspended fusion polypeptide for 10 min to 40 hours, preferably for 5 to 35 hours, more preferable for 5 to 24 hours, most preferably for 8 to 16 hours, at 15 to 85°C, preferably at 30 to 60°C, more preferably at 40 to 55°C, for cleaving the target peptide from the fusion polypeptide by nickel-induced cleavage of the amino acid sequence X^{a}SX^{b}X^{c}X^{d}X^{e}X^{f}X^{g};
and wherein step d) comprises the sub-steps of
d1) precipitating the purification domain and cleavage domain by adding Ca²⁺, preferably Ca²⁺-containing buffer, and
d2) isolating the target peptide.

17. The method according to claim 13 or 14,
wherein step a) includes providing the fusion polypeptide in inclusion bodies;
wherein step b) comprises the sub-steps of
b1) solubilising the inclusion bodies in Ni²⁺-containing buffer, and
b2) incubating the solubilized fusion polypeptide for 10 min to 40 hours, preferably for 5 to 35 hours, more preferable for 5 to 24 hours, most preferably for 8 to 16 hours, at 15 to 85°C, preferably at 30 to 60°C, more preferably at 40 to 55°C, for cleaving the target peptide from the fusion polypeptide by nickel-induced cleavage of the amino acid sequence X^{a}SX^{b}X^{c}X^{d}X^{e}X^{f}X^{g};
wherein step c) comprises refolding the purification domain and cleavage domain as well as the target peptide obtained in step b2) in Ca²⁺containing buffer, thereby precipitating the purification domain and cleavage domain; and
wherein step d) comprises isolating the target peptide.
